# EUROPEAN PATENT SPECIFICATION

(11) **EP 2 008 573 B1**
(45) Date of publication and mention of the grant of the patent: **31.05.2017**
(21) Application number: 07741357.3
(22) Date of filing: 10.04.2007
(51) Int. Cl.: A61B 1/04, A61B 1/00, H04N 7/18

(54) **BIOLOGICAL OBSERVATION SYSTEM**
BIOLOGISCHES BETRACHTUNGSSYSTEM
SYSTÈME D'OBSERVATION BIOLOGIQUE

(30) Priority: 20.04.2006 JP 2006117052
(43) Date of publication of application: 31.12.2008
(73) Proprietor: Olympus Corporation, Tokyo 192-8507 (JP)
(72) Inventor: YAMAZAKI, Kenji, Shibuya-ku Tokyo 151-0072 (JP); GONO, Kazuhiro, Shibuya-ku Tokyo 151-0072 (JP)
(74) Representative: von Hellfeld, Axel
(86) International application number: PCT/JP2007/057921
(87) International publication number: WO 2007/123028

(56) References cited:
- EP-A1- 1 374 755
- JP-A- 2002 095 635
- JP-A- 2002 345 733
- JP-A- 2005 074 034
- JP-A- 2006 116 153
- JP-A- 2007 020 727
- JP-A- 2007 075 445
- US-A1- 2005 020 879
- US-B1- 6 471 636

## Description

### Technical Field

The present invention relates to a living body observation system and particularly to a living body observation system that can display two observation images together on the same display means.

### Background Art

An endoscope system having an endoscope, a light source apparatus, and the like has conventionally been widely used in the medical field and the like. Particularly, the endoscope system in the medical field is mainly used in applications in which an operator and the like perform observation and the like in a living body as an examinee.

Also, observation generally known as observation using an endoscope system in the medical field includes, for example, in addition to normal observation in which a subject in a living body is irradiated with a white light, and in which the image of the subject substantially similar to that in observation by the naked eye is picked up, narrow band imaging (NBI) in which the subject is irradiated with a narrow band light that is a light having a narrower band than an illumination light in normal observation to perform observation to pick up an image in which a blood vessel and the like in a mucosal surface layer in the living body is emphasized compared with those in normal observation.

An endoscope system proposed in Japanese Patent Application Laid-Open Publication No. 2002-095635 is configured by having a light source apparatus, in which a filter having discrete spectral characteristics is provided, for outputting an illumination light having a narrow band, and an endoscope for picking up the image of a subject illuminated by the illumination light. By having the above-described configuration, the endoscope system proposed in Japanese Patent Application Laid-Open Publication No. 2002-095635 can perform narrow band imaging on the subject.

However, the endoscope system proposed in Japanese Patent Application Laid-Open Publication No. 2002-095635 has a configuration in which only one of the image of a subject picked up in normal observation and the image of the subject picked up in narrow band imaging is displayed on a monitor. Therefore, when the endoscope system proposed in Japanese Patent Application Laid-Open Publication No. 2002-095635 is used, a situation can occur in which, for example, when performing screening for picking out a lesion, an operator must frequently perform operation for switching the observation content of the endoscope system to either normal observation or narrow band imaging, while performing operation and the like for inserting or withdrawing the endoscope.

Also, the endoscope system proposed in Japanese Patent Application Laid-Open Publication No. 2002-095635 is configured by having a mechanism for switching band limitation means provided in the light source apparatus with the change of observation content, and the like to enable normal observation and narrow band imaging. Therefore, a problem that a configuration for performing normal observation and narrow band imaging is complicated occurs in the endoscope system proposed in Japanese Patent Application Laid-Open Publication No. 2002-095635.

US 6 471 636 B1 , which is considered to represent the most relevant prior art and forms the basis for the preamble of claim 1, discloses a fluorescence diagnosis endoscope system comprising an electronic endoscope and a video processing unit. A light source, a rotary filter, a motor and a timing circuit are provided in the video processing unit. The processing unit is further provided with a video circuit for processing normal colour image, a video circuit for processing the fluorescence light image, an image integrating circuit and an image synthesizing circuit. The video circuit processes only the image signal output by a second image receiving element. The image synthesizing circuit received an integrated fluorescence light video signal from the image integrating unit and the colour video signals from the normal video circuit. In accordance with the operating status of the display switch, the image synthesizing circuit outputs the normal colour video signal or the fluorescence image video signal on the display unit. Based on the output signal of the timing circuit, the first and the second image receiving elements and are driven synchronously with the rotation of the motor and accordingly the RGB frame-sequential image reading is performed. The video signal detects the image signal when the object is illuminated with blue light. Since the filter is provided in front of the image receiving element, a fluorescence image video signal is output by the video circuit.

EP 1 374 755 A1 discloses an image processing device for fluorescence observation, by which different body parts like blood vessels, diseased tissue or normal tissue are coloured with different colours.

US 2005/020879 A1 discloses a spatial filter to be used in an electronic endoscope device.

The present invention has been made in view of the above-described points and aims to provide a living body observation system that can perform normal observation and narrow band imaging together without performing complicated operation and by a simple configuration.

### Disclosure of Invention

### Means for Solving the Problem

The invention is defined in claim 1.

### Brief Description of the Drawings

Fig. 1 is a view showing one example of a configuration of a main portion of a living body observation system according to the present embodiment;
Fig. 2 is a view showing the configuration of a rotary filter provided in the light source apparatus of the living body observation system in Fig. 1;
Fig. 3 is a view showing spectral characteristics of the R filter, G filter, and B filter of the rotary filter in Fig. 2;
Fig. 4 is a view showing the spectral characteristics of the B1 filter of the rotary filter in Fig. 2;
Fig. 5 is a view showing one example of a normal observation image and a narrow band imaging image displayed on a monitor of the living body observation system shown in Fig. 1;
Fig. 6 is a view showing one example of amplitude characteristics of the spatial filter of a filtering circuit in Fig. 1;
Fig. 7 is a view showing an example of the configuration of the main portion of the living body observation system according to the present embodiment, different from the example in Fig. 1;
Fig. 8 is a view showing an example of the configuration of the rotary filter provided in the light source apparatus of the living body observation system in Fig. 1, different from the example in Fig. 2; and
Fig. 9 is a view showing the spectral characteristics of the B1 filter and Gn filter of the rotary filter in Fig. 8.

### Best Mode for Carrying Out the Invention

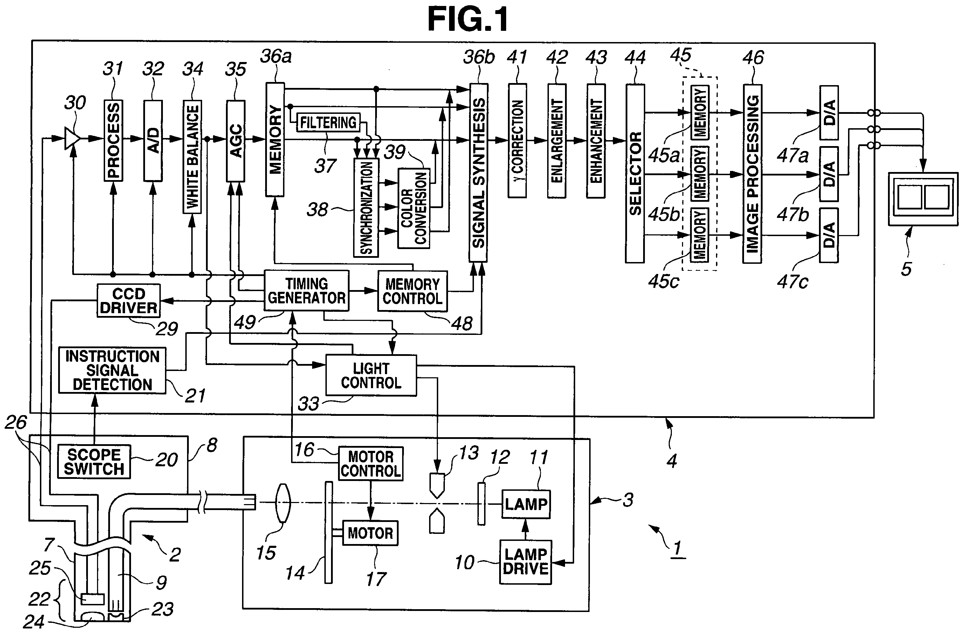

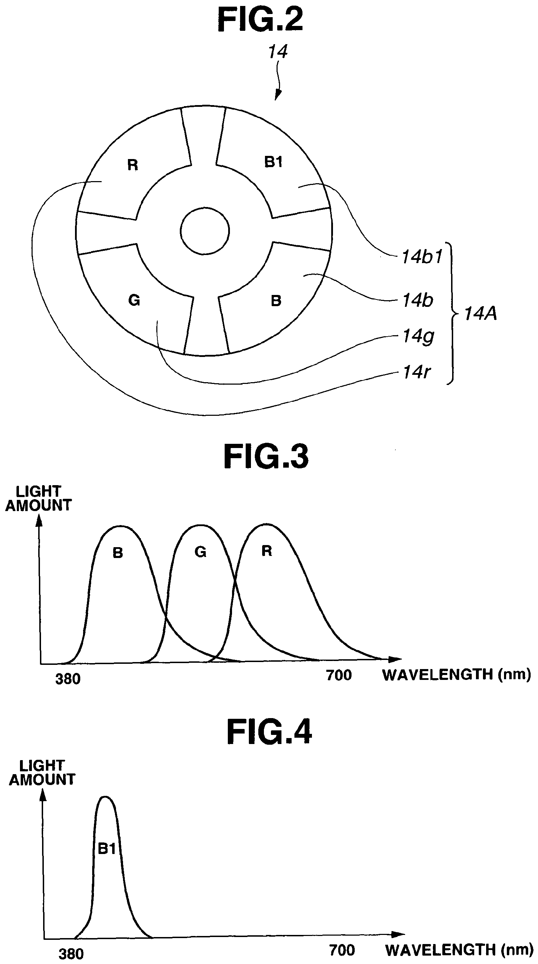

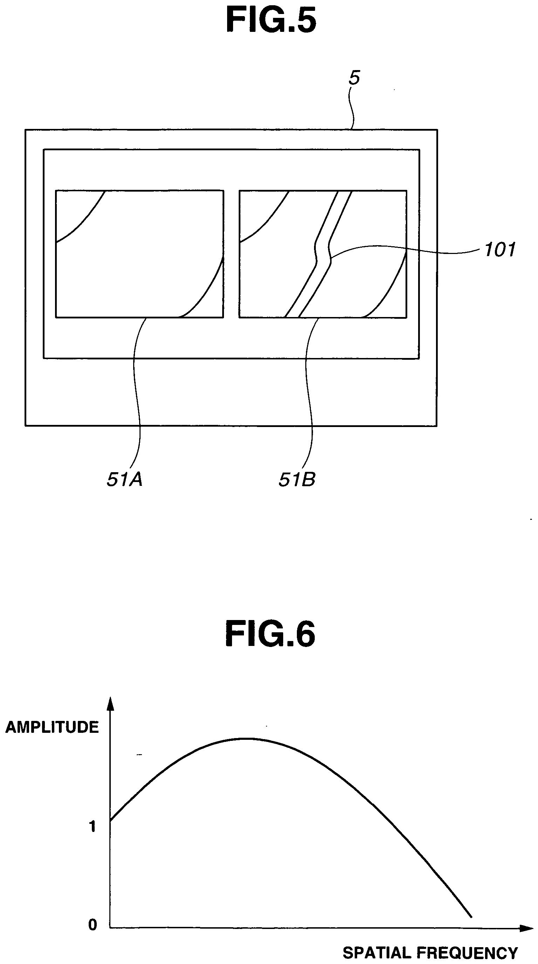

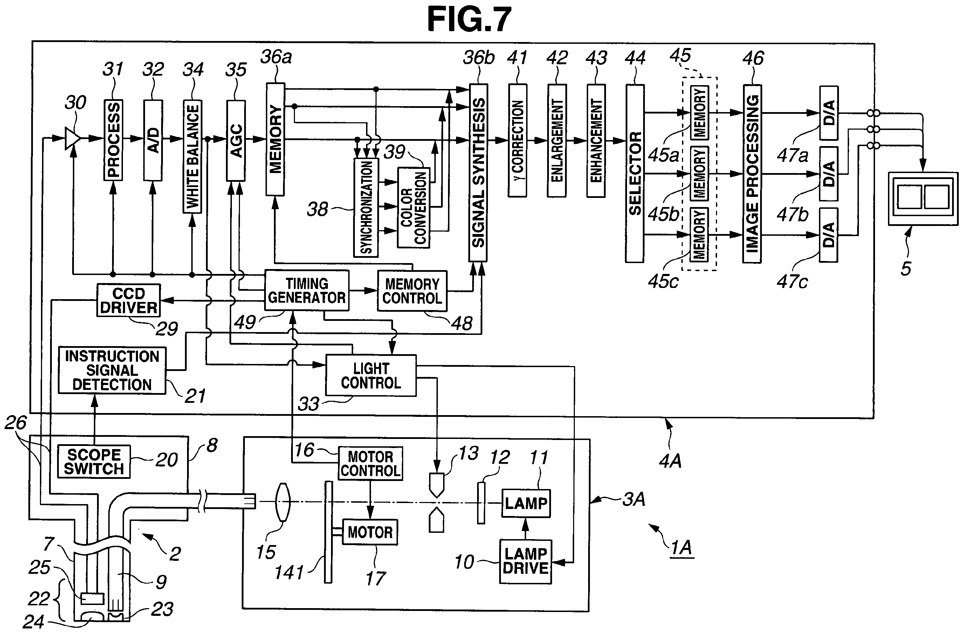

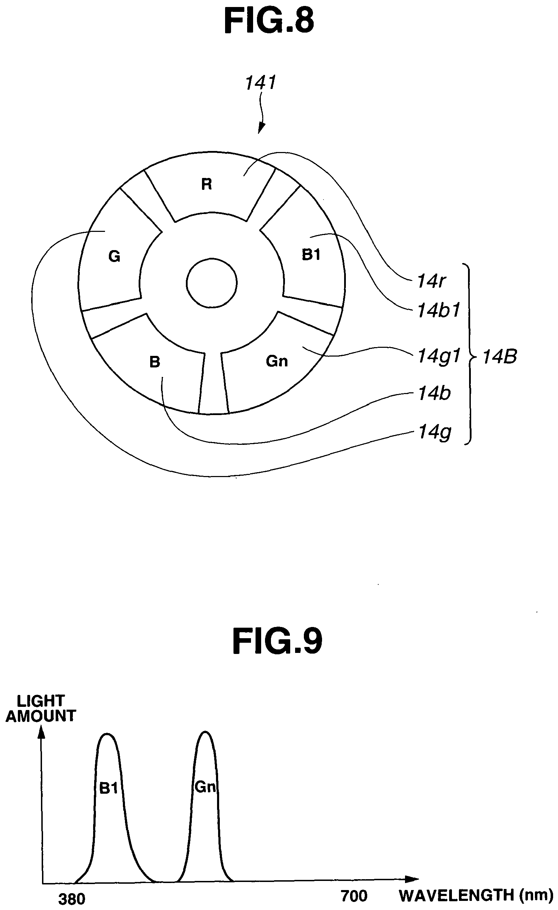

Fig. 1 to Fig. 9 relate to an embodiment of the present invention. Fig. 1 is a view showing one example of a configuration of a main portion of a living body observation system according to the present embodiment. Fig. 2 is a view showing the configuration of a rotary filter provided in the light source apparatus of the living body observation system in Fig. 1. Fig. 3 is a view showing spectral characteristics of the R filter, G filter, and B filter of the rotary filter in Fig. 2. Fig. 4 is a view showing the spectral characteristics of the B1 filter of the rotary filter in Fig. 2. Fig. 5 is a view showing one example of a normal observation image and a narrow band imaging image displayed on a monitor of the living body observation system shown in Fig. 1. Fig. 6 is a view showing one example of amplitude characteristics of the spatial filter of a filtering circuit in Fig. 1. Fig. 7 is a view showing an example of the configuration of the main portion of the living body observation system according to the present embodiment, different from the example in Fig. 1. Fig. 8 is a view showing an example of the configuration of the rotary filter provided in the light source apparatus of the living body observation system in Fig. 1, different from the example in Fig. 2. Fig. 9 is a view showing the spectral characteristics of the B1 filter and Gn filter of the rotary filter in Fig. 8.

The main portion of a living body observation system 1 is configured by having a living body image pickup apparatus 2, an endoscope or the like, that is inserted into a body cavity, picks up the image of a subject, such as a living tissue, in the body cavity, and outputs the image as an image pickup signal, a light source apparatus 3 that emits light for illuminating the subject to the living body image pickup apparatus 2, a video processor 4 that drives image pickup means included in the living body image pickup apparatus 2 and performs signal processing on the image pickup signal outputted from the living body image pickup apparatus 2 to output the signal as a video signal, and a monitor 5, as display means, that image-displays the image of the subject, based on the video signal outputted from the video processor 4, as shown in Fig. 1.

The living body image pickup apparatus 2 is configured by having an elongated insertion portion 7 that is inserted into a body cavity, and an operation portion 8 that is provided at the rear end of the insertion portion 7. The insertion portion 7 is configured by having a distal end portion 22 on the distal end side.

Also, the living body image pickup apparatus 2 has a scope switch 20 including one switch or a plurality of switches for giving various instructions, for example, an instruction to set the display mode of an image displayed on the monitor 5, to the video processor 4 by the operation of an operator or the like. Various instructions made in the scope switch 20 are outputted as instruction signals to the video processor 4.

The living body observation system 1 of the present embodiment has at least three display modes as the display mode that can be set in the scope switch 20. For example, there are a normal observation image including an image substantially similar to an image when a desired subject in a living body is observed by the naked eye, and a narrow band imaging image including an image in which the contrast of the image of a blood vessel present in the mucosal surface layer of the desired subject and a layer slightly deeper than the mucosal surface layer is enhanced. The system has at least three display modes, a combined observation mode in which the normal observation image and the narrow band imaging image are displayed together in one image, a normal observation mode in which only the normal observation image is displayed, and a narrow band imaging mode in which only the narrow band imaging image is displayed.

The distal end portion 22 of the living body image pickup apparatus 2 is configured by having an illumination lens 23 that is attached to an illumination window not shown, an objective lens 24 that is attached to an observation window, not shown, provided adjacent to the illumination window, and a CCD (charge coupled device) 25 that is an image pickup device located at the image formation position of the objective lens 24. Also, the CCD 25, as image pickup means, picks up the image of a subject formed by the objective lens 24, and outputs the picked up image of the subject as an image pickup signal. The image pickup signal outputted from the CCD 25 is outputted to the video processor 4 via a signal line 26. Also, the signal line 26 has a configuration that can be detachably connected to the video processor 4 via a connector not shown.

Also, a light guide 9 for transmitting light emitted from the light source apparatus 3 is inserted through the insertion portion 7. The light guide 9 has a configuration in which one end having a light exit surface is located on the light entrance side of the illumination lens 23 and in which the other end having a light entrance surface can be detachably connected to the light source apparatus 3.

The light source apparatus 3 has a lamp drive circuit 10 that is driven based on the control of a light control circuit 33 provided in the video processor 4, a lamp 11 that is driven based on drive current applied by the lamp drive circuit 10, a heat ray cut filter 12 that cuts off the heat rays of light emitted by the lamp 11, and an aperture apparatus 13 that controls the light amount of light emitted via the heat ray cut filter 12.

Also, the light source apparatus 3 has a rotary filter 14 that is located in the light path of the lamp 11 and converts light emitted from the aperture apparatus 13, as aperture means, to a frame sequential light to be able to be emitted, a condensing lens 15 that condenses light emitted from the rotary filter 14 and emits the light to the light entrance surface of the light guide 9, a motor control circuit 16, and a motor 17 that rotation-drives the rotary filter 14, based on the control of the motor control circuit 16.

The lamp 11, as light source means, is configured, for example, by a xenon lamp or the like and emits a white light including at least the band of a visible region. Also, based on drive current applied by the lamp drive circuit 10, the lamp 11 that is configured as a part of illumination means emits the white light with a light amount according to the current.

The rotary filter 14, as band limitation means, that is configured as a part of the illumination means is a disk-shaped filter with a center as a rotation axis and is configured by having a group of filters 14A in a circumferential portion, as shown in Fig. 2.

A group of filters 14A, as spectral means, is configured by having, as the spectral means, an R filter 14r that mainly transmits light in a red band, a G filter 14g that mainly transmits light in a green band, and a B filter 14b that mainly transmits light in a blue band, each of which are set to have spectral characteristics shown in Fig. 3, and further having, as the spectral means, a B1 filter 14b1 that is set to have the spectral characteristics of transmitting light in a band narrower than that for the B filter 14b, shown in Fig. 4.

Also, the motor control circuit 16 controls the rotation drive of the motor 17, and at timing according to the rotation drive, outputs a motor drive signal that is a signal used in the generation of a timing signal in a timing generator 49 provided in the video processor 4.

The motor 17 rotates the rotary filter 14 at a predetermined rotation speed, for example, 15 rotations per second, by rotation drive based on the control of the motor control circuit 16. By the motor control circuit 16 and the motor 17 having the above-described configuration, each filter of the group of filters 14A is sequentially interposed in the light path of the lamp 11.

The lamp drive circuit 10, as light amount control means, that is configured as a part of the illumination means applies drive current having a first current value to the lamp 11, based on the control of the light control circuit 33 provided in the video processor 4, at timing when the B1 filter 14b1 of the group of filters 14A is interposed in the light path of the lamp 11. Also, the lamp drive circuit 10 applies drive current having a second current value that is a current value smaller than the first current value to the lamp 11, based on the control of the light control circuit 33 provided in the video processor 4, at timing when each filter of the group of filters 14A, other than the B1 filter 14b1, is interposed in the light path of the lamp 11.

Based on an instruction signal outputted from the scope switch 20, an instruction signal detection circuit 21 provided in the video processor 4 outputs to a signal synthesis circuit 36b a control signal for displaying an image according to each display mode of the above-described combined observation mode, normal observation mode, and narrow band imaging mode.

When the rotary filter 14 rotates by the rotation drive of the motor 17 as drive means, the white light emitted from the lamp 11 is sequentially separated by being transmitted through the R filter 14r, G filter 14g, B filter 14b, and B1 filter 14b1 that are the filters of the group of filters 14A, condensed by the condensing lens 15, and then, sequentially enters the light entrance surface of the light guide 9.

The light emitted from the light source apparatus 3 enters the light entrance surface of the light guide 9, and then is emitted to a subject, such as a living tissue, via the illumination lens 23 provided on the light exit surface side.

The subject illuminated by light transmitted through the R filter 14r, light transmitted through the G filter 14g, light transmitted through the B filter 14b, and light transmitted through the B1 filter 14b1 that are sequentially emitted from the illumination lens 23 is image-formed by the objective lens 24, and then each image is picked up by the CCD 25. Then, the image of the subject picked up by the CCD 25 is outputted as an image pickup signal to the video processor 4 via the signal line 26.

The CCD 25 is connected to a CCD driver 29 that outputs a CCD drive signal to the CCD 25 at timing determined based on a timing signal outputted from a timing generator 49, and to a preamplifier 30. By such a configuration, the CCD 25 is driven based on the CCD drive signal outputted from CCD driver 29, and in a driven state, generates an image pickup signal and outputs the generated image pickup signal to the preamplifier 30.

The image pickup signal outputted from the CCD 25, as image pickup means, to the video processor 4, is amplified by the preamplifier 30, subjected to correlated double sampling, noise removal, and the like by a process circuit 31, converted to a digital signal by an A/D conversion circuit 32, and then inputted to a white balance circuit 34, at timing determined based on the timing signal outputted from the timing generator 49.

The white balance circuit 34 performs white balance processing on the inputted image pickup signal and then outputs the image pickup signal, after the white balance processing is performed, to the light control circuit 33 and an automatic gain control circuit (hereinafter abbreviated as AGC circuit) 35. Specifically, as the white balance processing, for example, when a white surface is a subject, the white balance circuit 34 calculates a white balance correction coefficient for each signal of the image pickup signal of the image of the subject picked up by the CCD 25 under light transmitted through the R filter 14r (hereinafter described as R signal), the image pickup signal of the image of the subject picked up by the CCD 25 under light transmitted through the B filter 14b (hereinafter described as B signal), and the image pickup signal of the image of the subject picked up by the CCD 25 under light transmitted through the B1 filter 14b1 (hereinafter described as B1 signal), based on the image pickup signal of the image of the subject picked up by the CCD 25 under light transmitted through the G filter 14g (hereinafter described as G signal), and multiplies the each signal by the white balance correction coefficient to perform processing that equalizes the intensity of the image pickup signals among the signals.

The AGC circuit 35 performs gain adjustment on the image pickup signal outputted from the white balance circuit 34, based on a brightness control signal outputted from the light control circuit 33 and the timing signal outputted from the timing generator 49, and outputs the image pickup signal after the gain adjustment to a memory 36a. Specifically, as the gain adjustment, for example, the AGC circuit 35 increases the gain of the B1 signal outputted from the white balance circuit 34 so that the B1 signal has one intensity.

Also, the timing generator 49 generates a timing signal for determining timing when each portion of the light source apparatus 3 and the video processor 4 performs processing, operation, and the like, based on a motor drive signal outputted from the motor control circuit 16, and outputs the timing signal to the each portion at predetermined timing.

A memory control circuit 48 performs control for outputting an image pickup signal stored in the memory 36a and the memory of the signal synthesis circuit 36b not shown to each portion at timing determined based on the timing signal outputted from the timing generator 49.

The memory 36a sequentially stores image pickup signals outputted from the AGC circuit 35, and based on the control of the memory control circuit 48, outputs image pickup signals inputted while the rotary filter 14 rotates once, to each portion, respectively. Specifically, the memory 36a outputs an R signal to the signal synthesis circuit 36b and a synchronization circuit 38 and outputs a G signal to the signal synthesis circuit 36b and a filtering circuit 37, based on the control of the memory control circuit 48. Also, the memory 36a outputs a B signal to the signal synthesis circuit 36b and outputs a B1 signal to the synchronization circuit 38, based on the control of the memory control circuit 48.

The filtering circuit 37 performs image enhancement processing for enhancing the low region to middle region frequency components of the G signal outputted from the memory 36a, so that the image of a subject including the image of a blood vessel present in a layer slightly deeper than a mucosal surface layer in a living body is image-displayed on the monitor 5 in a state in which the contrast of the image of the blood vessel is enhanced, and the filtering circuit 37 outputs the G signal, after the processing is performed, as a G1 signal to the synchronization circuit 38. Specifically, as the image enhancement processing, the filtering circuit 37 performs filtering processing using a spatial filter having the characteristics of transmitting the low region to middle region frequency components of the image of the subject based on the G signal outputted from the memory 36a. By the filtering circuit 37 performing the filter processing, the contrast of the image of the blood vessel present in the layer slightly deeper than the mucosal surface layer is enhanced. The filtering circuit 37 of the present embodiment is configured, for example, as one that performs the filtering processing using a spatial filter having amplitude characteristics as shown in Fig. 6.

The synchronization circuit 38 synchronizes the R signal and B1 signal outputted from the memory 36a, and the G1 signal outputted from the filtering circuit 37, and outputs the synchronized R signal, G1 signal, and B1 signal to a color conversion circuit 39.

The color conversion circuit 39 performs color conversion processing on the R signal, G1 signal, and B1 signal that are image pickup signals synchronized by the synchronization circuit 38 and outputted, for example, by using a 3 x 3 matrix, and outputs the R signal, G1 signal, and B1 signal, after the color conversion processing is performed, to the signal synthesis circuit 36b.

The signal synthesis circuit 36b is configured by having a memory not shown, and stores in the memory a first image pickup signal including the R signal, G signal, and B signal outputted from the memory 36a, and a second image pickup signal including the R signal, G1 signal, and B1 signal outputted from the color conversion circuit 39. Then, the signal synthesis circuit 36b, as first and second image generation means, generates an RGB signal according to a display mode set in the scope switch 20, from the first image pickup signal and the second image pickup signal, based on the control signal outputted from the instruction signal detection circuit 21, and control performed by the memory control circuit 48, and then sequentially outputs the R component, G component, and B component of the RGB signal to a γ correction circuit 41.

Specifically, for example, when the display mode of the image is set to the combined observation mode in the scope switch 20, by performing processing, for example, reduction processing, on each of a first R image that is the image of the subject according to the R signal of the first image pickup signal, and a second R image that is the image of the subject according to the R signal of the second image pickup signal, the signal synthesis circuit 36b outputs an image, in which the first R image is located on the left side in the image of one frame and in which the second R image is located on the right side in the image of one frame, as the R component in the RGB signal. Also, for example, when the display mode of the image is set to the combined observation mode in the scope switch 20, by performing processing, for example, reduction processing, on each of a G image that is the image of the subject according to the G signal of the first image pickup signal, and a G1 image that is the image of the subject according to the G1 signal of the second image pickup signal, the signal synthesis circuit 36b outputs an image, in which the G image is located on the left side in the image of one frame and in which the G1 image is located on the right side in the image of one frame, as the G component in the RGB signal. Further, for example, when the display mode of the image is set to the combined observation mode in the scope switch 20, by performing processing; for example, reduction processing, on each of a B image that is the image of the subject according to the B signal of the first image pickup signal, and a B1 image that is the image of the subject according to the B1 signal of the second image pickup signal, the signal synthesis circuit 36b outputs an image, in which the B image is located on the left side in the image of one frame and in which the B1 image is located on the right side in the image of one frame, as the B component in the RGB signal.

In other words, the signal synthesis circuit 36b generates an image, in which a reduced normal observation image and a narrow band imaging image are respectively located on left and right sides, as the image according to the combined observation mode, by performing each processing described above.

Also, for example, when the display mode of the image is set to the normal observation mode in the scope switch 20, the signal synthesis circuit 36b outputs the first R image as the R component in the RGB signal, outputs the G image as the G component in the RGB signal, and outputs the B image as the B component in the RGB signal.

Further, for example, when the display mode of the image is set to the narrow band imaging mode in the scope switch 20, the signal synthesis circuit 36b outputs the second R image as the R component in the RGB signal, outputs the G1 image as the G component in the RGB signal, and outputs the B1 image as the B component in the RGB signal.

Each component of the RGB signal outputted from the signal synthesis circuit 36b is γ-corrected by the γ correction circuit 41, subjected to enlargement and interpolation processing by an enlargement circuit 42, and then inputted to an enhancement circuit 43.

The enhancement circuit 43 performs structure enhancement or contour enhancement processing on each component of the RGB signal outputted from the enlargement circuit 42 and then outputs the RGB signal, after the processing is performed, to a selector 44.

Then, the RGB signal outputted from the enhancement circuit 43 is inputted to a synchronization circuit 45 via the selector 44.

The synchronization circuit 45 is configured by having three memories 45a, 45b, and 45c for storing each component of the RGB signal outputted from the selector 44. The synchronization circuit 45 synchronizes and outputs each component of the RGB signal stored in the memories 45a, 45b, and 45c.

The RGB signal synchronized in the synchronization circuit 45 and outputted is subjected to image processing, such as the color shift correction of a moving image, by an image processing circuit 46 and then inputted to D/A conversion circuits 47a, 47b, and 47c.

The D/A conversion circuits 47a, 47b, and 47c store each component of the RGB signal outputted from the image processing circuit 46, convert the stored each component to an analog video signal, and then output the video signal to the monitor 5.

Based on the intensity of the B1 signal outputted from the white balance circuit 34, the light control circuit 33 outputs a brightness control signal for increasing the intensity of the B1 signal to one intensity to the AGC circuit 35. Also, based on the intensity of each signal of the R signal, G signal, and B signal outputted from the white balance circuit 34, the light control circuit 33 performs control over the aperture apparatus 13 so that the intensity of the each signal is a predetermined intensity. Further, the light control circuit 33 performs control for outputting drive current while switching the current value of the drive current, according to timing when each filter of the group of filters 14A is interposed in the light path of the lamp 11, over the lamp drive circuit 10, based on the timing signal outputted from the timing generator 49. By performing each control as described above over the lamp drive circuit 10, the aperture apparatus 13, and the AGC circuit 35, the light control circuit 33 adjusts the brightness of an image when the image of the subject picked up by the living body image pickup apparatus 2 is displayed as the image on the monitor 5.

Next, the operation of the living body observation system 1 of the present embodiment will be described.

First, an operator or the like connects the living body image pickup apparatus 2 to the light source apparatus 3 and the video processor 4 in a state as shown in Fig. 1 and turns on the power of the each portion and the monitor 5 to activate the living body observation system 1. Immediately after activation, the scope switch 20 is set to the combined observation mode.

When the living body observation system 1 is activated, the motor control circuit 16 controls the rotation drive of the motor 17 so that the rotary filter 14 located in the light path of the lamp 11 rotates at a predetermined rotation speed, such as 15 rotations per second. Also, the motor control circuit 16 outputs a motor drive signal to the timing generator 49 at timing according to the predetermined rotation speed. The timing generator 49 generates a timing signal for determining timing when each portion of the light source apparatus 3 and the video processor 4 performs processing, operation, and the like, based on the motor drive signal outputted from the motor control circuit 16, and outputs the timing signal to the each portion at predetermined timing. Then, the CCD driver 29 of the video processor 4 outputs a CCD drive signal to the CCD 25, based on the timing signal outputted from the timing generator 49.

Also, when the living body observation system 1 is activated, the light control circuit 33 of the video processor 4 performs control for outputting drive current while switching the current value of the drive current, according to timing when each filter of the group of filters 14A is interposed in the light path of the lamp 11, over the lamp drive circuit 10, based on a timing signal outputted from the timing generator 49. Also, the lamp drive circuit 10 applies drive current to the lamp 11 while alternately switching drive current having a first current value and drive current having a second current value, based on the control of the light control circuit 33.

Consequently, the lamp 11 emits a white light having a relatively large light amount according to drive current having the first current value, at timing when the B1 filter 14b1 of the group of filters 14A is interposed in the light path of the lamp 11. Also, the lamp 11 emits a white light having a relatively small light amount according to drive current having the second current value, at timing when the each filter of the group of filters 14A, other than the B1 filter 14b1, is interposed in the light path of the lamp 11. As a result, the S/N of a B1 signal is improved, and the contrast in a narrow band imaging image displayed on the monitor 5 is enhanced.

By the rotation of the rotary filter 14 associated with the rotation drive of the motor 17, a white light emitted in the lamp 11 is sequentially separated by being transmitted through the R filter 14r, G filter 14g, B filter 14b, and B1 filter 14b1 that are the filters of the group of filters 14A. Then, the light transmitted through each filter of the group of filters 14A is condensed by the condensing lens 15, and then, sequentially enters the light entrance surface of the light guide 9 as an illumination light.

Each illumination light transmitted after entering the light guide 9 is sequentially emitted to a subject via the illumination lens 23.

The CCD 25 is driven based on the CCD drive signal outputted from the CCD driver 29, picks up the image of the subject that is illuminated by each illumination light sequentially emitted from the illumination lens 23 and is further image-formed by the objective lens 24, and outputs the picked up image of the subject to the video processor 4 as an image pickup signal.

The image pickup signal outputted from the CCD 25 to the video processor 4 is amplified by the preamplifier 30, subjected to correlated double sampling, noise removal, and the like by the process circuit 31, converted to a digital signal by the A/D conversion circuit 32, subjected to white balance processing by the white balance circuit 34, subjected to gain adjustment by the AGC circuit 35, and then outputted to the memory 36a, at timing determined based on the timing signal outputted from the timing generator 49.

As the gain adjustment, the AGC circuit 35 increases the gain of a B1 signal outputted from the white balance circuit 34 so that the B1 signal has one intensity, based on a brightness control signal outputted from the light control circuit 33 and the timing signal outputted from the timing generator 49. In other words, the B1 signal outputted from the white balance circuit 34 is outputted to the memory 36a, always having the one intensity, by passing through the AGC circuit 35.

Also, the memory control circuit 48 performs control for outputting an image pickup signal stored in the memory 36a and the memory of the signal synthesis circuit 36b not shown to each portion at timing determined based on the timing signal outputted from the timing generator 49.

The memory 36a sequentially stores image pickup signals outputted from the AGC circuit 35, and based on the control of the memory control circuit 48, outputs image pickup signals inputted while the rotary filter 14 rotates once, to the signal synthesis circuit 36b, filtering circuit 37, and synchronization circuit 38, respectively.

An R signal, a G signal, and a B signal directly outputted from the memory 36a to the signal synthesis circuit 36b are stored in the memory of the signal synthesis circuit 36b, not shown, as signals constituting a first image pickup signal, until a second image pickup signal is inputted to the signal synthesis circuit 36b.

An R signal and a B1 signal directly outputted from the memory 36a to the synchronization circuit 38, and a G1 signal that is a G signal outputted from the memory 36a and then subjected to the above-described image enhancement processing by the filtering circuit 37 are synchronized by the synchronization circuit 38, subjected to color conversion processing by the color conversion circuit 39, and then outputted as a second image pickup signal, which is stored in the signal synthesis circuit 36b. The second image pickup signal is not limited to one including the R signal, G1 signal, and B1 signal, and may be, for example, one including only the G1 signal and B1 signal.

Based on a control signal outputted from the instruction signal detection circuit 21, and the control of the memory control circuit 48, the signal synthesis circuit 36b generates an RGB signal according to the combined observation mode, from the first image pickup signal and second image pickup signal stored in the memory not shown, and sequentially outputs the R component, G component, and B component of the RGB signal to the γ correction circuit 41.

Each component of the RGB signal outputted from the signal synthesis circuit 36b is γ-corrected by the γ correction circuit 41, subjected to enlargement and interpolation processing by the enlargement circuit 42, subjected to structure enhancement or contour enhancement processing by the enhancement circuit 43, and then inputted to the synchronization circuit 45 via the selector 44.

Then, the synchronization circuit 45 stores each component of the RGB signal outputted from the selector 44 and synchronizes and outputs the each component.

The RGB signal synchronized in the synchronization circuit 45 and outputted is subjected to image processing, such as the color shift correction of a moving image, by the image processing circuit 46 and then inputted to the D/A conversion circuits 47a, 47b, and 47c.

The D/A conversion circuits 47a, 47b, and 47c store each component of the RGB signal outputted from the image processing circuit 46, convert the stored each component to an analog video signal, and then output the video signal to the monitor 5.

By processing and the like as described above being performed in the video processor 4, for example, an image substantially similar to an image when a desired subject in a living body is observed by the naked eye, and an image in which the contrast of the image of a blood vessel 101 present in the mucosal surface layer of the desired subject and a layer slightly deeper than the mucosal surface layer is enhanced are displayed together on the same screen of the monitor 5 as a normal observation image 51 A and a narrow band imaging image 51B, respectively, as shown in Fig. 5.

As described above, the living body observation system 1 of the present embodiment has a configuration that is capable of the displaying normal observation image 51 A and the narrow band imaging image 51B together on the same screen of the monitor 5.

Therefore, by using the living body observation system 1, an operator or the like can perform normal observation and narrow band imaging together, while looking at a normal observation image and a narrow band imaging image displayed on the same screen of the same monitor, without performing complicated operation.

Further, in the living body observation system 1 of the present embodiment, two or more band limitation means are not provided in the light source apparatus. Therefore, the living body observation system 1 of the present embodiment does not need a mechanism for switching the band limitation means provided in the light source apparatus with the change of observation content, or the like, and as a result, the living body observation system 1 of the present embodiment is capable of performing normal observation and narrow band imaging by a configuration simpler than conventional one.

Also, in the normal observation mode of the living body observation system 1 of the present embodiment, an illumination light having substantially the same spectral characteristics as that in a conventional living body observation system, such as an endoscope apparatus, is emitted to a subject, and a video signal having the image of the subject according to the illumination light is generated. Therefore, in the living body observation system 1 of the present embodiment, as a normal observation image, an image having substantially the same color tone as that in a conventional living body observation system, such as an endoscope apparatus, or an image in a state in which substantially the same color reproduction as that in a conventional living body observation system, such as an endoscope apparatus, is achieved is generated. Consequently, also when the operator performs normal observation using the living body observation system 1 of the present embodiment, instead of a conventional living body observation system, such as an endoscope apparatus, the operator can perform observation without feeling uncomfortable.

The rotary filter 14, as the band limitation means, in the present embodiment may be one having a configuration other than that of a rotary filter, as long as a configuration in which each light of light transmitted through the R filter 14r, the light transmitted through the G filter 14g, light transmitted through the B filter 14b, and light transmitted through the B1 filter 14b1 can be sequentially generated is achieved. Also, the rotary filter 14 may be one located anywhere in the light path from the light emitting side of the lamp 11 to the image pickup surface of the CCD 25.

Also, the living body observation system 1 of the present embodiment is not limited to one having the above-described configuration and may be, for example, one configured as a living body observation system 1A as shown in Fig. 7.

The main portion of the living body observation system 1A is configured by having an endoscope 2, a light source apparatus 3A in which a rotary filter 141 is provided instead of the rotary filter 14 in the light source apparatus 3, a video processor 4A having a configuration similar to a configuration in which a filtering circuit 37 is removed from the video processor 4, and a monitor 5.

The rotary filter 141 of the light source apparatus 3A has a group of filters 14B in a circumferential portion, as shown in Fig. 8.

The group of filters 14B is configured by further having a Gn filter 14g1 in the circumferential portion, in addition to an R filter 14r, a G filter 14g, a B filter 14b, and a B1 filter 14b1, as the filters of a group of filters 14A. Also, the Gn filter 14g1 is set to have spectral characteristics of transmitting light in a band narrower than that for the G filter 14g, as shown in Fig. 9.

Here, the operation of the living body observation system 1A will be described.

The image pickup signal of the image of a subject picked up by the CCD 25 under light transmitted through the Gn filter 14g1 (hereinafter described as Gn signal) is amplified by the preamplifier 30, subjected to correlated double sampling, noise removal, and the like by the process circuit 31, converted to a digital signal by the A/D conversion circuit 32, subjected to white balance processing by the white balance circuit 34, subjected to gain adjustment by the AGC circuit 35, and then inputted to the memory 36a.

The memory 36a sequentially stores image pickup signals outputted from the AGC circuit 35, and based on the control of the memory control circuit 48, outputs image pickup signals inputted while the rotary filter 141 rotates once, to each portion, respectively. Specifically, the memory 36a outputs an R signal to the signal synthesis circuit 36b and the synchronization circuit 38 and outputs a G signal to the signal synthesis circuit 36b, based on the control of the memory control circuit 48. Also, the memory 36a outputs a B signal to the signal synthesis circuit 36b and outputs a B1 signal and a Gn signal to the synchronization circuit 38, based on the control of the memory control circuit 48.

The R signal, B1 signal, and Gn signal outputted from the memory 36a are synchronized by the synchronization circuit 38, subjected to color conversion processing by the color conversion circuit 39, and then outputted as a third image pickup signal, which is stored in the signal synthesis circuit 36b.

Then, the third image pickup signal is subjected to processing similar to the above-described processing for the second image pickup signal in each portion of the signal synthesis circuit 36b and portions downstream of the signal synthesis circuit 36b of the video processor 4A. Consequently, the image of the blood vessel 101 present in the mucosal surface layer of a desired subject and a layer slightly deeper than the mucosal surface layer is image-displayed on the monitor 5 of the living body observation system 1A, with contrast higher than that in a case where the living body observation system 1 is used.

## Claims

1. A living body observation system (1) comprising:
illumination means (3) adapted to sequentially emit as an illumination light broad band lights of a red light, a green light and a first blue light, and a narrow band light in a blue region having a narrower wavelength band than the broad band lights to a subject in a living body to illuminate the subject;
image pickup means (2) adapted to pick up each return light from the subject when the broad band lights and the narrow band light in the blue region are sequentially emitted from the illumination means (3), signal processing means (4) adapted to generate a first observation image based on signals of each colour component obtained by picking up the return lights of the broad band lights, **characterized by** the signal processing means further adapted to generate a second observation image based on a signal obtained by enhancing frequency components from a low region to a middle region of a green component signal obtained by picking up the return light of the green light, and a signal of a narrow band component obtained by picking up a second blue light which is the return light of the narrow band light in the blue region, wherein a circuit (36b) is adapted to
combine the first observation image and the second observation image to generate a combined image, and wherein the circuit (36b) is adapted to
input a signal representing the combined image into an enhancement circuit (43) to perform enhancing processing for structure enhancement or contour enhancement on the combined image.

2. The living body observation system (1) according to claim 1, wherein the illumination means (3) has light source means (11) adapted to emit a white light, and band limitation means (14) that is located in a light path from the light source means (11) to the image pickup means (2) and limits a wavelength band of the white light to sequentially separate the white light into the red light, the green light, the first blue light, and the narrow band light in the blue region.

3. The living body observation system (1) according to claim 2, wherein the band limitation means (14) is configured as a rotary filter (24) which has spectral means (14A) adapted to separate the white light emitted in the light source means (11) to generate the red light, the green light and the first blue light and the narrow band light in the blue region, and in which by rotation associated with rotation drive by drive means (12), the spectral means (14A) is sequentially interposed in the light path of the light source means (11).

4. The living body observation system (1) according to claim 1, wherein the image enhancement processing for enhancing frequency components from a low region to a middle region of the green component signal is filtering processing using a spatial filter.

5. The living body observation system according to any one of claims 1-4, wherein the illumination means (3) has light amount control means (10) adapted to control a light amount of each light sequentially emitted as an illumination light.

## Patentansprüche

1. System (1) zum Beobachten eines lebendigen Körpers, umfassend:
Beleuchtungsmittel (3), die geeignet sind, um als Beleuchtungslicht breitbandige Lichter von einem roten Licht, einem grünen Licht und einem ersten blauen Licht und ein schmalbandiges Licht in einer blauen Region, das ein schmaleres Wellenlängenband als die breitbandigen Lichter aufweist, auf ein Motiv in einem lebendigen Leib sequenziell zu emittieren, um das Motiv zu beleuchten;
Bildaufnahmemittel (2), die geeignet sind, um jedes zurückkehrende Licht von dem Motiv aufzunehmen, wenn die breitbandigen Lichter und das schmalbandige Licht in der blauen Region von den Beleuchtungsmitteln (3) sequenziell emittiert werden,
Signalverarbeitungsmittel (4), die geeignet sind, um ein erstes Betrachtungsbild basierend auf Signalen jeder Farbkomponente, die durch Aufnehmen der zurückkehrenden Lichter der breitbandigen Lichter erzielt wird, zu generieren
**dadurch gekennzeichnet, dass** die Signalverarbeitungsmittel ferner geeignet sind, um ein zweites Betrachtungsbild basierend auf einem Signal, das durch das Verstärken von Frequenzkomponenten aus einer niedrigen Region auf eine mittlere Region eines Grünkomponentensignals erzielt wird, indem das zurückkehrende Licht des grünen Lichts aufgenommen wird, und einem Signal einer schmalbandigen Komponente, das erzielt wird, indem ein zweites blaues Licht aufgenommen wird, wobei es sich um das zurückkehrende Licht des schmalbandigen Lichts in der blauen Region handelt, zu generieren, wobei eine Schaltung (36b) geeignet ist, um das erste Betrachtungsbild und das zweite Betrachtungsbild zu kombinieren, um ein kombiniertes Bild zu generieren, und wobei die Schaltung (36b) geeignet ist, um ein Signal, welches das kombinierte Bild darstellt, in eine Verstärkungsschaltung (43) einzugeben, um eine Verstärkungsverarbeitung für eine Strukturverstärkung oder Umrissverstärkung an dem kombinierten Bild vorzunehmen.

2. System (1) zum Beobachten eines lebendigen Körpers nach Anspruch 1, bei dem das Beleuchtungsmittel (3) Lichtquellenmittel (11), die geeignet sind, um ein weißes Licht zu emittieren, und ein Bandbegrenzungsmittel (14), das sich auf einem Lichtweg von dem Lichtquellenmittel (11) bis zu dem Bildaufnahmemittel (2) befindet und ein Wellenlängenband des weißen Lichts begrenzt, um das weiße Licht der Reihe nach in das rote Licht, das grüne Licht, das erste blaue Licht und das schmalbandige Licht in der blauen Region zu trennen, aufweist.

3. System (1) zum Beobachten eines lebendigen Körpers nach Anspruch 2, bei dem das Bandbegrenzungsmittel (14) als ein Rotationsfilter (24) konfiguriert ist, das Spektralmittel (14A) aufweist, die geeignet sind, um das weiße Licht, das in dem Lichtquellenmittel (11) emittiert wird, zu trennen, um das rote Licht, das grüne Licht und das erste blaue Licht und das schmalbandige Licht in der blauen Region zu generieren, und in dem durch eine Drehung, die mit dem Drehantrieb durch die Antriebsmittel (12) verknüpft ist, das Spektralmittel (14A) sequenziell auf dem Lichtweg des Lichtquellenmittels (11) eingeschoben ist.

4. System (1) zum Beobachten eines lebendigen Körpers nach Anspruch 1, bei dem die Bildverstärkungsverarbeitung zum Verstärken von Frequenzkomponenten aus einer niedrigen Region in eine mittlere Region des Grünkomponentensignals eine Filterverarbeitung unter Verwendung eines räumlichen Filters ist.

5. System (1) zum Beobachten eines lebendigen Körpers nach einem der Ansprüche 1 bis 4, bei dem das Beleuchtungsmittel Lichtmengenregelmittel (10) aufweist, die geeignet sind, um eine Lichtmenge jedes Lichts, das sequenziell als Beleuchtungslicht emittiert wird, zu regeln.

## Revendications

1. Système d'observation de corps vivant (1) comprenant :
des moyens d'éclairage (3) adaptés pour émettre de manière séquentielle, en tant que lumière d'éclairage, des lumières à large bande d'une lumière rouge, d'une lumière verte et d'une première lumière bleue, et une lumière à bande étroite dans une région bleue ayant une bande de longueur d'onde plus étroite que les lumières à large bande, sur un sujet dans un corps vivant pour éclairer le sujet ;
des moyens de capture d'image (2) adaptés pour capturer chaque lumière de retour provenant du sujet lorsque les lumières à large bande et la lumière à bande étroite dans la région bleue sont émises de manière séquentielle à partir des moyens d'éclairage (3),
des moyens de traitement de signal (4) adaptés pour générer une première image d'observation sur la base de signaux de chaque composante de couleur obtenus par capture des lumières de retour des lumières à large bande,
**caractérisé par** les moyens de traitement de signal en outre adaptés pour générer une seconde image d'observation sur la base d'un signal obtenu par amélioration de composantes de fréquence d'une région basse à une région intermédiaire d'un signal de composante de vert obtenu par capture de la lumière de retour de la lumière verte, et d'un signal d'une composante de bande étroite obtenu par capture d'une seconde lumière bleue qui est la lumière de retour de la lumière à bande étroite dans la région bleue, un circuit (36b) étant adapté pour
combiner la première image d'observation et la seconde image d'observation pour générer une image combinée, et le circuit (36b) étant adapté pour
entrer un signal représentant l'image combinée dans un circuit d'amélioration (43) pour réaliser un traitement d'amélioration pour une amélioration de structure ou une amélioration de contour sur l'image combinée.

2. Système d'observation de corps vivant (1) selon la revendication 1, dans lequel les moyens d'éclairage (3) ont des moyens de source de lumière (11) adaptés pour émettre une lumière blanche, et des moyens de limitation de bande (14) qui sont situés dans un trajet de lumière allant des moyens de source de lumière (11) aux moyens de capture d'image (2) et limitent une bande de longueur d'onde de la lumière blanche pour séparer de manière séquentielle la lumière blanche en la lumière rouge, la lumière verte, la première lumière bleue, et la lumière à bande étroite dans la région bleue.

3. Système d'observation de corps vivant (1) selon la revendication 2, dans lequel les moyens de limitation de bande (14) sont configurés sous la forme d'un filtre rotatif (24) qui a des moyens spectraux (14A) adaptés pour séparer la lumière blanche émise dans les moyens de source de lumière (11) pour générer la lumière rouge, la lumière verte et la première lumière bleue et la lumière à bande étroite dans la région bleue, et dans lequel, par rotation associée à un entraînement en rotation par des moyens d'entraînement (12), les moyens spectraux (14A) sont interposés de manière séquentielle dans le trajet de lumière des moyens de source de lumière (11).

4. Système d'observation de corps vivant (1) selon la revendication 1, dans lequel le traitement d'amélioration d'image pour améliorer des composantes de fréquence d'une région basse à une région intermédiaire du signal de composante de vert est un traitement de filtrage utilisant un filtre spatial.

5. Système d'observation de corps vivant selon l'une quelconque des revendications 1 à 4, dans lequel les moyens d'éclairage (3) ont des moyens de commande de quantité de lumière (10) adaptés pour commander une quantité de lumière de chaque lumière émise de manière séquentielle comme lumière d'éclairage.
